# EUROPEAN PATENT APPLICATION

(11) **EP 4 644 528 A1**
(43) Date of publication of application: **05.11.2025**
(21) Application number: 22871044.8
(22) Date of filing: 28.12.2022
(51) Int. Cl.: C12M 3/00, C12M 1/00, C12M 1/12, C12M 1/42

(54) **BIOREACTOR CHAMBER FOR GROWING AND/OR MATURING A TISSUE AND METHOD FOR GROWING AND/OR MATURING A TISSUE IN A BIOREACTOR CHAMBER**

(71) Applicant: LEARTIKER S.COOP., 48270 Markina-Xemein (Bizkaia) (ES); Universidad de Navarra, 31009 Pamplona (Navarra) (ES)
(72) Inventor: GEREKA GOIENETXE, Ainitze, 20808 GETARIA (GIPUZKOA) (ES); ZALDUA HUICI, Ane Miren, 20006 DONOSTIA (GIPUZKOA) (ES); LARREATEGI MAKATZAGA, Pablo, 48270 MARKINA-XEMEIN (BIZKAIA) (ES); PAGALDAY AZPIRI, Rikardo, 48270 MARKINA-XEMEIN (BIZKAIA) (ES); PRÓSPER CARDOSO, Felipe Luis, 31008 PAMPLONA (NAVARRA) (ES); MAZO VEGA, Manuel Maria, 31008 PAMPLONA (NAVARRA) (ES); IGLESIAS GARCÍA, Olalla, 31008 PAMPLONA (NAVARRA) (ES)
(74) Representative: Galbaian S.Coop.
(86) International application number: PCT/ES2022/070839
(87) International publication number: WO 2024/141677

(57) **Abstract**

Bioreactor chamber for growing and/or maturing a tissue comprising a container (1) for containing a culture medium and securing means (2) configured to secure inside the container a flexible support (S) that is permeable to the culture medium in which cells that will form the tissue are arranged. The chamber further comprises mechanical stimulation means (3) which generate a cyclical movement of the support (S) by means of a pressure surface (4) which pushes the support (S) by intermittently contacting said support (S). Method for growing and/or maturing a tissue in a bioreactor chamber.

## Description

### TECHNICAL FIELD

The present invention relates to bioreactor chambers for growing and/or maturing a tissue and to methods for growing and/or maturing a tissue in a bioreactor chamber.

### PRIOR ART

Bioreactor chambers for growing and/or maturing a tissue, maintaining cells that will form the tissue in a culture medium under controlled conditions, are known. In many cases, maintaining the cells in a culture medium is not sufficient and requires stimulation in order to induce vital functions in the cells that will form the tissue.

Bioreactor chambers for growing and/or maturing a tissue which also comprise mechanical stimulation in order to induce vital functions in the cells that will form the tissue, for example, a heart tissue or lung tissue are known.

For example, WO2015108869A2 discloses a bioreactor chamber for growing a heart tissue comprising a container for containing a culture medium, securing means configured to secure inside the container a flexible support that is permeable to the culture medium in which cells that will form the tissue are arranged, and mechanical stimulation means which generate a cyclical movement of the support. The chamber is configured for the mechanical stimulation means to act on the culture medium, which generates the cyclical movement of the support. It also discloses a method for growing a heart tissue in said chamber.

### DISCLOSURE OF THE INVENTION

The object of the invention is to provide a bioreactor chamber for growing and/or maturing a tissue and a method for growing and/or maturing a tissue in a bioreactor chamber as defined in the claims.

A first aspect of the invention relates to a bioreactor chamber for growing and/or maturing a tissue comprising a container for containing a culture medium and securing means configured to secure inside the container a flexible support that is permeable to the culture medium in which cells that will form the tissue are arranged. The chamber further comprises mechanical stimulation means which generate a cyclical movement of the support by means of a pressure surface which pushes the support by intermittently contacting said support.

A second aspect of the invention relates to a bioreactor comprising a chamber such as the one described above.

A third aspect of the invention relates to a method for growing and/or maturing a cell tissue in a bioreactor chamber. The method comprises the steps of securing a flexible support that is permeable to the culture medium in which the cells that will form the tissue in the chamber are arranged and generating a cyclical movement of the support by means of mechanical stimulation means, in which said cyclical movement is performed by pushing the support with a pressure surface which intermittently contacts said support.

The support is thereby acted on by contact of a pressure surface, whereby enabling more precise control over the deformation applied to the cells that will form the tissue while maintaining the cells in the culture medium in one and the same container.

These and other advantages and features of the invention will become apparent in view of the figures and detailed description of the invention.

### DESCRIPTION OF THE DRAWINGS

Figure 1 is an exploded view of a bioreactor chamber according to an embodiment of the invention.
Figure 2 is a sectioned perspective view of the chamber of Figure 1 in a standby position.
Figure 3 is a schematic depiction of the cyclical movement in the chamber of Figure 1.
Figure 4 is a sectioned front view of the container and of the lid of the chamber of Figure 1.
Figure 5 is a sectioned perspective view of the securing means of the chamber of Figure 1.
Figure 6 is a sectioned perspective view of the securing means of a bioreactor chamber according to another embodiment of the invention.

### DETAILED DISCLOSURE OF THE INVENTION

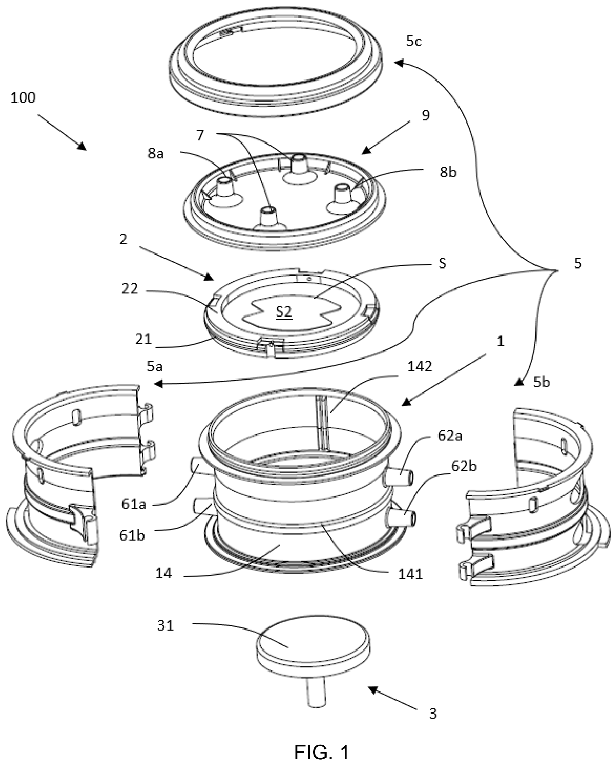

Figures 1 to 3 show a bioreactor chamber 100 for growing and/or maturing a tissue comprising a container 1 for containing a culture medium and securing means 2 configured to secure inside the container a flexible support S that is permeable to the culture medium in which cells that will grow and form the tissue are arranged. The chamber further comprises mechanical stimulation means 3 which generate a cyclical movement of the support S by means of a pressure surface 4 which pushes the support S by intermittently contacting said support S.

The chamber of the invention thereby allows the cells deposited on the support to better imitate the physiological conditions experienced by the tissue *in vivo.* By pushing the support through intermittent contact of a pressure surface, the deformation applied to the cells that will form the tissue while the cells are kept submerged in the culture medium in one and the same container can be more precisely controlled.

In the context of the invention, support refers to a scaffold, construct, or membrane on which the deposited cells are supported in order to grow and form the tissue. The support can be manufactured with materials of a natural origin, such as collagen, alginate, hyaluronic acid, gelatin, fibrin, biopolymers such as PCL, PLA, PGA, PLA-PEG, PLGA, decellularized matrix, or a combination thereof. It can be manufactured with structures of a different pore size or with structures that can be adapted to each tissue. The support is usually biocompatible and inert with respect to the medium, gradually degradable with cell growth and tissue formation, of a porosity suitable for cell attachment, and its derivatives are not toxic.

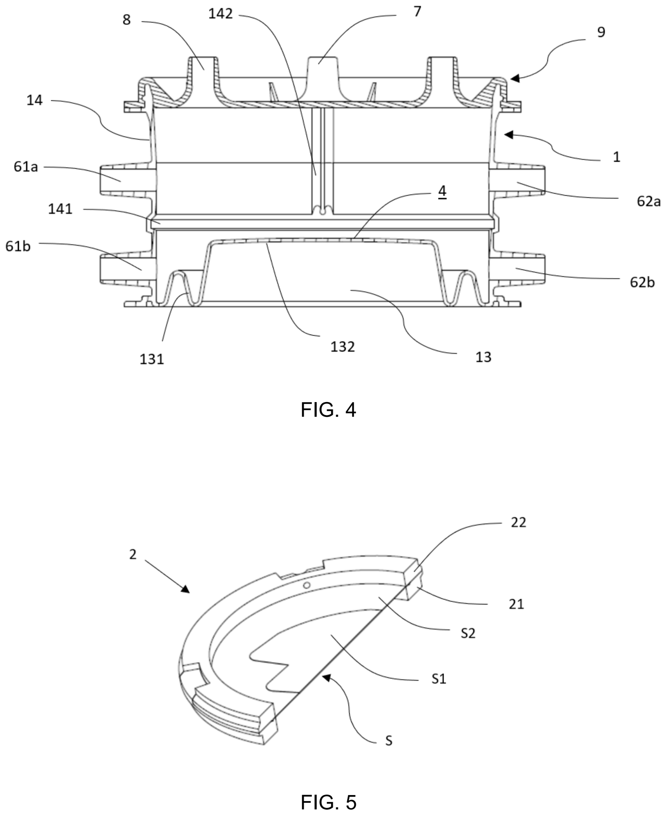

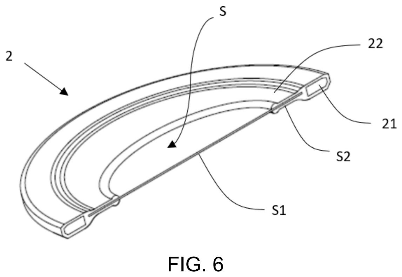

The support S of the invention is flexible and permeable to the culture medium, therefore, the support S is deformed when the pressure surface 4 pushes the support S while the securing means 2 secure it. The support S comprises a regenerative area S1 in which the cells are deposited and which is pushed by the pressure surface 4, and a fixing area S2 which is secured at least in part by the securing means 2, as shown in Figures 5 and 6.

A cyclical movement of the support in the context of the invention refers to a movement that is repeated in cycles and in which in each cycle, the support is deformed, returning to the initial position and, accordingly, the cells arranged on the support are also deformed cyclically. Said cyclical movements allow the cells that will form the tissue, for example, the heart tissue, to be subjected to deformations intended to simulate the actual movement of the tissue in a live organ. The cyclical movement thereby allows imitating the physiological conditions experienced by the tissue in the future implantation in vivo.

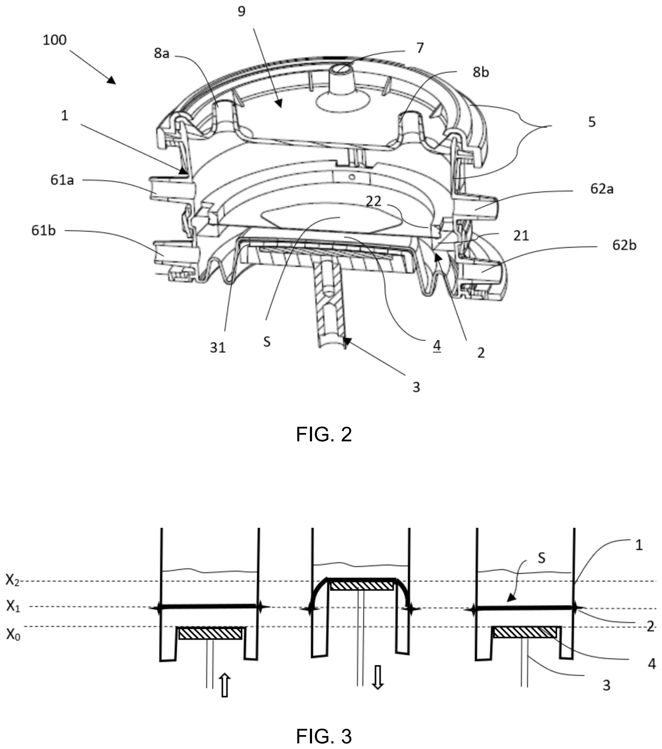

Figure 3 schematically shows the cyclical movement generated in the bioreactor chamber 100 shown in Figures 1 and 2. In a standby position X₀, with this standby position being the position in which the mechanical stimulation means 3 do not act, the pressure surface 4 and the support S are not in contact. The mechanical stimulation means 3 move, starting from and returning to said standby position X₀ in each cycle, and with said movement of the mechanical stimulation means 3, the pressure surface 4 pushes the support S by intermittently contacting said support S. In order to actually push the support S with the pressure surface 4 in each cycle, the mechanical stimulation means 3 move the pressure surface 4 past a threshold position X₁, with this threshold position X₁ being the position in which the pressure surface 4 actually contacts the support S.

The culture medium provides the nutrients needed by the cells to grow when the cells are kept submerged in said culture medium.

In the context of the invention, intermittently contacting refers to the fact that the pressure surface contacts and ceases to contact the support in each cycle instead of being in continuous contact, allowing, on one hand, the nutrients to reach all the cells in an easier fashion, and on the other hand, a gas exchange so that the cells have enough oxygen supply, thereby reducing the risk of tissue necrosis due to a lack of nutrients and oxygen.

The securing means 2 keep the support S submerged in the culture medium even with said cyclical movement so that the cells arranged on the support S can be kept viable and grow during the tissue formation and stimulation process, which can last for several months depending on the types of cells used.

In a preferred embodiment, the support S is a membrane. In this way, since the regenerative area of the support is a substantially two-dimensional area, it is easier for the pressure surface to act on the entire regenerative area of the support, performing a mechanically more homogenous stimulation in the cells arranged on the support.

In a preferred embodiment, the pressure surface 4 is a deformable wall of the container 1 arranged between the mechanical stimulation means 3 and the securing means 2, as occurs in the preferred embodiment shown in Figures 1 to 3. In this way, since the mechanical stimulation means are arranged outside the container, the introduction of foreign elements into the container that may affect the sterility of the culture medium and of the cells arranged on the support, and the need for additional components to ensure the leak-tightness are prevented.

However, in another embodiment not shown in the figures, the pressure surface can be a surface of the mechanical stimulation means arranged inside the container.

In a preferred embodiment, the container 1 is formed by a single deformable part. A leak-tight container with a simple and easy-to-manufacture and easy-to-assemble container construction is thereby obtained.

The container 1 is preferably made of an elastomeric material, more preferably silicone. The high elasticity of elastomeric materials allows the deformable wall of the container to be deformed and move until it contacts and pushes the support, deforming only elastically when the mechanical stimulation means act, and it can recover its original shape until it ceases to contact the support when the mechanical stimulation means cease to act. Among the most elastic elastomeric materials, silicone stands out, although the container can also be made of other elastomeric materials such as TPU, TPE or EVA.

In a preferred embodiment, the container 1 is covered by a rigid casing 5 which partially covers the container 1, immobilizing the securing means 2 inside the container 1. The casing 5 can be made of metal, a composite, or a rigid polymer. In this way, the securing means remain immobile inside the container, even though a portion of the container, such as the deformable wall, is deformed.

The casing 5 can be formed by several parts which, once assembled, immobilize the securing means 2 inside the container 1. To that end, the parts can have a shape complementary to the portions of the container 1 covered by these parts.

In a preferred embodiment, the securing means 2 are configured to generate an auxotonic stimulation of the support S. Auxotonic stimulation refers to a type of contraction in which the cells or tissue being generated on the flexible and permeable support try to overcome the resistance to radial elongation of an elastomeric material.

In a preferred embodiment, the securing means 2 comprise a first and a second securing element 21 and 22 between which the perimetral edge of the support S is secured, with the first securing element 21 preferably being a ring. The perimetral edge of the support is thereby secured, while the central area of the support can be for arranging cells without the securing means being able to damage them and for the pressure surface to be able to push the support by intermittently contacting said support. That is, the fixing area S2 of the support S is located at the perimetral edge, and the regenerative area S1 of the support S is located in the central area of the support S.

In a preferred embodiment, the second securing element 22 is a second ring or a wall of the container 1. A better securing against mechanical stimulation is thereby obtained.

In another preferred embodiment the second securing element 22 is a covering made of an elastomeric material which covers the first securing element 21, as shown in Figure 6. Said securing means 2 are particularly advantageous for generating an auxotonic stimulation in the cells that will form the tissue arranged on the support S.

In the securing means 2 shown in Figure 6, the first securing element 21 is a ring made of a rigid material and the second securing element 22 is a covering made of an elastomeric material, such as silicone, which covers the first securing element 21 and the perimetral edge of the support S. Said securing means 2 are particularly advantageous for generating an auxotonic stimulation in the cells that will form the tissue arranged on the support S, by securing the previously radially stretched support S with the silicone covering. The support S tries to overcome the resistance to radial elongation exerted by the securing means 2, causing a contraction force on said support S and on the cells. In this way, in addition to allowing mechanical stimulation to be exerted, the securing means allow an auxotonic stimulation to be exerted on the tissue formed by the cells.

The securing means 2 can be detachably arranged inside the container 1. In this way, the securing means of the container together with the support can be easily removed and put back into place as many times as desired, allowing the tissue to be analyzed without having to touch it at any time during its growth or allowing the tissue to be taken out of the container without breaking once its growth is complete so that it can be implanted in the patient.

In a preferred embodiment, the mechanical stimulation means 3 are configured to exert different contact pressures on the support S by means of the pressure surface 4. In this way, it allows the degree of deformation of the support S to be adjusted according to the degree of deformation required throughout cell growth.

The mechanical stimulation means 3 may comprise an actuator 31 with a set of interchangeable heads, with the surface contour of each head being different. When the mechanical stimulation means 3 act, the actuator 31 pushes the pressure surface 4 which is deformed, adopting the surface contour of the head used in each case, in such a way that with each head used different contact pressures can be exerted on the support S depending on the degree of deformation required throughout cell growth.

The mechanical stimulation means 3 preferably comprise a linear actuator 31 which moves vertically and is arranged below the container 1, as shown in Figures 1 to 3.

The chamber 100 may comprise at least one medium inlet port 61 and one medium outlet port 62 which are connected with the inside of the container 1 to recirculate the culture medium. In this way, a continuous supply and control of nutrients over long periods for cell growth and tissue formation can be ensured without the need to schedule stops for renewal of the culture medium in the container.

In a preferred embodiment, the chamber 100 comprises a first and a second culture medium inlet port 61a and 61b, and a first and a second culture medium outlet port 62a and 62b. The first inlet port 61a and the first outlet port 62a are arranged above the securing means 2, and the second inlet port 61b and the second outlet port 62b are arranged below the securing means 2, such that the culture medium can recirculate above and below the support. The risk of cell necrosis due to a lack of nutrient regeneration on one side of the support is thereby reduced.

In a preferred embodiment, the chamber 100 comprises at least one electrode port 7 through which electrodes are inserted into the container 1 for electrical stimulation of the cells. In this way, the cells can be stimulated both mechanically and electrically in one and the same chamber. The container 1 may comprise guide rails 142 along which the electrodes slide when inserted through the electrode port 7 to guide and fix the electrodes inside the container 1.

Additionally, the chamber 100 may comprise at least one gas inlet port 8a and at least one gas outlet port 8b connected with the inside of the container 1 to recirculate a gas in the container 1. In this way, it allows a gas supply and control of the optimal conditions of said continuous gas supply over long periods of growth and external to the chamber, without the need to put the chamber inside another device such as, for example, an incubator with which the optimal conditions of gas concentration, temperature, and pressure inside the container 1 for tissue growth are controlled.

In addition to the container 1, the chamber 100 may comprise a lid 9 for closing the container 1. In this way, it is easier to reach and maintain optical conditions of gas concentration, temperature, and pressure inside the container 1 for tissue growth.

The lid 9 can be deformable like the container 1. In this way, the lid 9 can also be deformed so as not to generate additional pressure inside the container 1 when the pressure surface 4 deforms.

Likewise, the lid 9 can be made of an elastomeric material like the container 1. In this way, the lid 9 is easily deformable, and once it is deformed, it can recover its original shape in the absence of external forces.

The lid 9 is preferably transparent optical grade silicone. In this way, the lid 9 facilitates the study of tissue growth and/or maturation in the chamber, since there is no need to open the lid 9 or remove the securing means 2 to measure the applied deformation or to analyze tissue growth and/or maturation parameters.

In a preferred embodiment, the lid 9 comprises the at least one electrode port 7 and/or the gas inlet and outlet ports 8a and 8b. In this way, the entry of some external elements into the chamber from above the container is focused, the construction of the container is simplified, and it allows to reduce the effect that the movement of the pressure surface may have on such external elements.

In the preferred embodiment shown in the figures, the lid 9 comprises two electrode ports 7 and the gas inlet and outlet ports 8a and 8b. From above, through each electrode port 7 of the lid 9 an electrode can be inserted until it contacts the support on which the cells are arranged so that electrical stimulation can be exerted directly on the cells or on the tissue once formed. The gas ports 8a and 8b allow the gases trapped in the container 1 to be recirculated between the lid 9 and the culture media.

One of the preferred embodiments of the chamber 100 shown in Figures 1 to 3 is described below.

With regard to the container 1, in this preferred embodiment the container 1 comprises a base 13 the inner wall of which comprises the pressure surface 4 and side walls 14 on which the securing means 2 are arranged, as shown in Figure 4.

The base 13 may comprise side folds 131 and a flat central portion 132 the inner wall of which is the pressure surface 4. In this way, the side folds allow the flat central portion of the base to be vertically moved so that it pushes the support when the mechanical stimulation means act on said base.

In this preferred embodiment, the container 1 is formed by a single deformable cylindrical part made of silicone and the base 13 has a circular shape, the side walls 14 constituting a single wall.

However, in other embodiments not shown in the figures, the container 1 and the base 13 can have other shapes, such as an oval, square, or rectangular shape.

With regard to the casing 5, in the preferred embodiment the casing 5 is formed by three parts 5a, 5b and 5c; the parts 5a and 5b of the casing 5 cover the side walls 14 of the container 1, while the part 5c of the casing 5 covers the edges of the container 1 and of the lid 9. In this way, the entire base and a large part of the lid can be deformed, while the side walls remain immobile and the closure of the container is ensured.

With regard to the securing means 2, in the preferred embodiment the first securing element 21 and the second securing element 22 of the securing means 2 are rings (see Figure 5), with the perimetral edge of the support S being secured between said rings, which are preferably made of a rigid material.

The side walls 14 of the container 1 may comprise a groove 141 for detachably arranging the securing means 2, said groove 141 being in a plane parallel to the plane of the flat central portion 132 of the base 13. To couple them, it is sufficient to lift the lid 9 and insert the securing means 2 from above the container 1 by deforming the side walls 14 until they fit into said groove 141, and they can be easily taken out again by pulling on them to uncouple same. Such a configuration makes it possible to secure the support on the side walls 14 submerged in the culture medium, dividing the container 1 into two sections.

With regard to the mechanical stimulation means 3, in the preferred embodiment, the mechanical stimulation means 3 comprise an actuator 31 which is linear and is arranged outside the container 1, below the base 13 of the container 1. The flat central portion 132 of the base 13 is supported on the actuator 31 and close to but separated from the support S in the standby position X₀, with this standby position being the position in which the mechanical stimulation means 3 do not act.

However, in other embodiments not shown in the figures the mechanical stimulation means may comprise an actuator which, in addition to allowing the pressure surface to intermittently contact the support, allows torsion to be exerted on the support.

With respect to the medium inlet and outlet ports 61a and 61b 62a and 62b, in the preferred embodiment these are arranged on the side walls 14 of the container 1, and the parts 5a and 5b of the casing 5 covering said side walls 14 comprise openings complementary to said medium ports 61a, 61b, 62a and 62b for them to pass through. In this way, the damage that the movement of the pressure surface may cause on the medium ports is reduced.

The invention also relates to a bioreactor for growing and/or maturing a tissue comprising the described chamber 100.

The bioreactor is preferably a bioreactor for growing and/or maturing a heart tissue. The use of the chamber of the invention in a bioreactor for growing and/or maturing a heart tissue allows a better imitation of the physiological conditions experienced by the tissue in vivo with respect to deformation of the heart muscle. In this way, the tissue will acquire structural and functional characteristics very similar to natural heart muscle tissue, which will in turn allow for a successful implantation in the patient.

Although a bioreactor for growing and/or maturing a heart tissue has been described in particular, the bioreactor according to the invention could also be for growing and/or maturing another type of tissue, such as skeletal muscle tissue, skin tissue, lung tissue, bladder tissue, cartilage tissue, or bone tissue.

Preferably, the container 1 and/or the securing means 2 are removable. The container 1 and/or the securing means 2 can be coupled to the rest of the components such that they can be easily removed from the bioreactor. In this way, it allows taking to the operating room where the tissue will be implanted only the securing means which secure the support, or the container together with the securing means which secure the support, simplifying the transfer and preventing the tissue from being able to be contaminated or from deteriorating during said transfer.

Another aspect of the invention relates to a method for growing and/or maturing a tissue in a bioreactor chamber, the method comprising the steps of:
- securing a flexible support that is permeable to the culture medium in which the cells that will form the tissue are arranged in the bioreactor chamber surrounded by culture medium, and
- generating a cyclical movement of the support by means of mechanical stimulation means, in which said cyclical movement is performed by pushing the support with a pressure surface which intermittently contacts said support.

In this way, the deformation applied to the cells that will form the tissue while the cells are kept in the culture medium in one and the same container can be more precisely controlled.

In a preferred embodiment of the method, an auxotonic stimulation of the support is generated during the step of securing. Auxotonic stimulation refers to a type of contraction in which the cells or tissue being generated on the flexible and permeable support try to overcome the resistance to radial elongation of an elastomeric material, such as that of the securing element 22 of the support S of Figure 6. In this way, the tissue will acquire a higher contractile force that is more similar to natural tissues, which in turn will allow a successful implantation in the patient.

In some cases, the auxotonic stimulation of the support during the step of securing can also be combined with electrical stimulation.

Said method can be used in a chamber 100 such as the one described above.

## Claims

1. Bioreactor chamber for growing and/or maturing a tissue, comprising a container (1) for containing a culture medium, securing means (2) configured to secure inside the container (1) a flexible support (S) that is permeable to the culture medium in which cells that will form the tissue are arranged, and mechanical stimulation means (3) which generate a cyclical movement of the support (S), **characterized in that** the mechanical stimulation means (3) generate the cyclical movement by means of a pressure surface (4) which pushes the support (S) by intermittently contacting said support (S).

2. Bioreactor chamber according to claim 1, wherein the support (S) is a membrane.

3. Bioreactor chamber according to claim 1 or 2, wherein the pressure surface (4) is a deformable wall of the container (1) arranged between the mechanical stimulation means (3) and the securing means (2).

4. Bioreactor chamber according to any of the preceding claims, wherein the container (1) is formed by a single deformable part.

5. Bioreactor chamber according to any of the preceding claims, wherein the container (1) is made of an elastomeric material, preferably silicone.

6. Bioreactor chamber according to any of the preceding claims, wherein the container (1) is covered by a rigid casing (5) which partially covers the container (1), immobilizing the securing means (2) inside the container (1).

7. Bioreactor chamber according to any of the preceding claims, wherein the securing means (2) are configured to generate an auxotonic stimulation of the support (S).

8. Bioreactor chamber according to any of the preceding claims, wherein the securing means (2) comprise a first and a second securing element (21, 22) between which the perimetral edge of the support (S) is secured, with the first securing element (21) preferably being a ring.

9. Bioreactor chamber according to claim 8, wherein the second securing element (22) is a second ring or a wall of the container (1).

10. Bioreactor chamber according to claim 9, wherein the second securing element (22) is a covering made of an elastomeric material which covers the first securing element (21).

11. Bioreactor chamber according to any of the preceding claims, wherein the securing means (2) are detachably arranged inside the container (1).

12. Bioreactor chamber according to any of the preceding claims, wherein the mechanical stimulation means (3) are configured to exert different contact pressures on the support (S) by means of the pressure surface (4).

13. Bioreactor chamber according to any of the preceding claims, comprising at least one medium inlet port (61) and one medium outlet port (62) which are connected with the inside of the container (1) to recirculate the culture medium.

14. Bioreactor chamber according to claim 13, comprising a first and a second culture medium inlet port (61a, 61b), and a first and a second culture medium outlet port (62a, 62b), with the first inlet port (61a) and the first outlet port (62a) being arranged above the securing means (2), and the second inlet port (61b) and the second outlet port (62b) being arranged below the securing means (2), such that the culture medium can recirculate above and below the support (S).

15. Bioreactor chamber according to any of the preceding claims, comprising at least one electrode port (7) through which electrodes are inserted into the container (1) for electrical stimulation of the cells.

16. Bioreactor chamber according to any of the preceding claims, comprising at least one gas inlet port (8a) and at least one gas outlet port (8b) connected with the inside of the container (1) to recirculate a gas in the container (1).

17. Bioreactor chamber according to any of the preceding claims, comprising a lid (9) for closing the container (1).

18. Bioreactor chamber according to claim 17, wherein the lid (9) comprises the at least one electrode port (7) and/or the gas inlet and outlet ports (8a, 8b).

19. Bioreactor chamber according to claim 17 or 18, wherein the lid (9) is deformable.

20. Bioreactor chamber according to any of claims 17 to 19, wherein the lid (9) is made of an elastomeric material, preferably transparent optical grade silicone.

21. Bioreactor chamber according to any of the preceding claims, wherein the container (1) comprises a base (13) the inner wall of which comprises the pressure surface (4) and side walls (14) in which the securing means (2) are arranged.

22. Bioreactor for growing and/or maturing a tissue comprising a chamber (100) according to any of the preceding claims.

23. Bioreactor according to the claim 22, wherein the tissue is a heart tissue.

24. Bioreactor according to claim 22 or 23, wherein the container (1) and/or the securing means (2) securing the support (S) are removable.

25. Method for growing and/or maturing a tissue in a bioreactor chamber, the method comprising the steps of:
- securing a flexible support that is permeable to the culture medium in which cells that will form the tissue are arranged in the bioreactor chamber surrounded by culture medium, and
- generating a cyclical movement of the support by means of mechanical stimulation means,
**characterized in that** the cyclical movement is performed by pushing the support with a pressure surface which intermittently contacts said support.

26. Method for growing and/or maturing a tissue according to claim 25, wherein an auxotonic stimulation of the support is generated during the step of securing.

27. Method for growing and/or maturing a tissue according to the claim 25 or 26, wherein the chamber is a chamber (100) according to any of the claims 1 to 21.
